# EUROPEAN PATENT APPLICATION

(11) **EP 4 840 328 A2**
(43) Date of publication of application: **18.11.2026**
(21) Application number: 26188222.9
(22) Date of filing: 06.11.2015
(51) Int. Cl.: A24B 15/167

(54) **SOLUTION**

(30) Priority: 07.11.2014 GB 201419865
(62) Document number(s) of the earlier application(s) in accordance with Art. 76 EPC: 20185947.7 / 3 747 282; 15794253.3 / 3 214 956
(71) Applicant: Nicoventures Trading Limited, London WC2R 3LA (GB)
(72) Inventor: MCADAM, Kevin Gerard, London, WC2R 3LA (GB); BRUTON, Connor, London, WC2R 3LA (GB)
(74) Representative: D Young & Co LLP

(57) **Abstract**

There is provided a nicotine solution comprising (i) a carrier; (ii) nicotine in unprotonated form and in protonated form; and (iii) benzoic acid.

## Description

### FIELD OF THE INVENTION

The present disclosure relates to a nicotine solution, containers in which are contained the nicotine solution and to electronic vapour provision systems such as electronic nicotine delivery systems (e.g. e-cigarettes) incorporating said solution.

### BACKGROUND TO THE INVENTION

Electronic vapour provision systems such as e-cigarettes generally contain a reservoir of liquid which is to be vaporised, typically containing nicotine. When a user inhales on the device, a heater is activated to vaporise a small amount of liquid, which is therefore inhaled by the user.

The use of e-cigarettes in the UK has grown rapidly, and it has been estimated that there are now over a million people using them in the UK.

One challenge faced in providing such systems is to provide from the vapour provision device a vapour to be inhaled which provides consumers with an acceptable experience.

Some consumers may prefer an e-cigarette that generates an aerosol that closely 'mimics' smoke inhaled from a tobacco product such as a cigarette. Aerosols from e-cigarettes and smoke from tobacco products such as cigarettes provides to the user a complex chain of flavour in the mouth, nicotine absorption in the mouth and throat, followed by nicotine absorption in the lungs. These various aspects are described by users in terms of flavour, intensity/quality, impact, irritation/smoothness and nicotine reward. Nicotine contributes to a number of these factors, and is strongly associated with factors such as impact, irritation and smoothness; these are readily perceived by consumers, and e-cigarettes may offer too much or too little of these parameters for consumers, depending upon individual preferences. Nicotine reward is particularly complex as it results from both the amount of and speed with which nicotine is absorbed from the lining of the mouth, this is typically nicotine in the vapour phase, and from the amount and speed nicotine that is absorbed from the lungs, this is typically nicotine in the particulate phase of the aerosol which is inhaled. Each of these factors, and their balance, can strongly contribute to consumer acceptability of an e-cigarette. Providing means to optimise the overall vaping experience is therefore desirable to e-cigarette manufacturers.

### SUMMARY OF THE INVENTION

In one aspect there is provided a nicotine solution comprising
(i) a carrier;
(ii) nicotine in unprotonated and in protonated form; and
(iii) one or more acids, wherein at least benzoic acid, levulinic acid or a mixture thereof is present; and
wherein the total content of acid present in the solution is no greater than 0.6 mole equivalents based on the nicotine.

In one aspect there is provided a contained nicotine solution comprising
(a) a container; and
(b) a nicotine solution, comprising
   (i) a carrier;
   (ii) nicotine in unprotonated form and in protonated form; and
   (iii) one or more acids, wherein at least benzoic acid, levulinic acid or a mixture thereof is present; and
   wherein the total content of acid present in the solution is no greater than 0.6 mole equivalents based on the nicotine.

In one aspect there is provided an electronic vapour provision system comprising:
a vaporiser for vaporising liquid for inhalation by a user of the electronic vapour provision system;
a power supply comprising a cell or battery for supplying power to the vaporiser a nicotine solution, comprising
   (i) a carrier;
   (ii) nicotine in unprotonated form and in protonated form; and
   (iii) one or more acids, wherein at least benzoic acid, levulinic acid or a mixture thereof is present; and
      wherein the total content of acid present in the solution is no greater than 0.6 mole equivalents based on the nicotine.

In one aspect there is provided a process for improving the sensory properties of a vaporised nicotine solution, the process comprising the steps of
(a) providing a nicotine solution comprising
   (i) a carrier;
   (ii) nicotine in unprotonated form and in protonated form; and
   (iii) one or more acids, wherein at least benzoic acid, levulinic acid or a mixture thereof is present; and
      wherein the total content of acid present in the solution is no greater than 0.6 mole equivalents based on the nicotine;
(b) vaporising the nicotine solution.

In one aspect there is provided use of one or more acids for improving sensory properties of a vaporised nicotine solution, wherein the nicotine solution comprises
(i) a carrier;
(ii) nicotine in unprotonated form and in protonated form; and
(iii) one or more acids, wherein at least benzoic acid, levulinic acid or a mixture thereof is present; and
wherein the total content of acid present in the solution is no greater than 0.6 mole equivalents based on the nicotine.

### DETAILED DESCRIPTION

As discussed herein the present invention provides a nicotine solution comprising (i) a carrier; (ii) nicotine in unprotonated form and in protonated form; and (iii) one or more acids, wherein at least benzoic acid, levulinic acid or a mixture thereof is present; and wherein the total content of acid present in the solution is no greater than 0.6 mole equivalents based on the nicotine.

We have found that by protonating some and only some of the nicotine present in a solution, such that the solution contains nicotine in unprotonated form and nicotine in protonated form, the solution when vaporised and inhaled provides desirable properties of flavour, impact, irritation, smoothness and/or nicotine reward for the user. We have particularly found that the levels of acid addition required by the present invention, namely wherein the total content of acid present in the solution is no greater than 0.6 mole equivalents based on the nicotine, may be used across a broad range of nicotine content solutions. At the levels of acid addition required by the present invention solutions may be provided having desirable properties of flavour, impact, irritation, smoothness and/or nicotine reward for the user both when the nicotine content is relatively low, such as 1.8 wt% nicotine or less and when the nicotine content is relatively high, such as greater than 1.8wt% nicotine.

As is understood by one skilled in the art, nicotine may exist in unprotonated form, monoprotonated form or diprotonated form. The structures of each of these forms are given below.

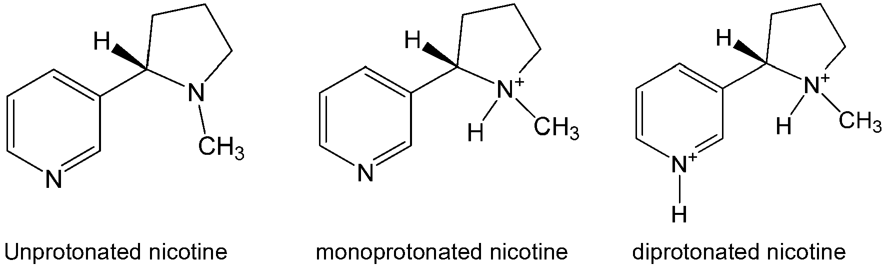

Reference in the specification to protonated form means both monoprotonated nicotine and diprotonated nicotine. Reference in the specification to amounts in the protonated form means the combined amount of monoprotonated nicotine and diprotonated nicotine. Furthermore, when reference is made to a fully protonated formulation it will be understood that at any one time there may be very minor amounts of unprotonated nicotine present, e.g. less than 1% unprotonated.

For ease of reference, these and further aspects of the present invention are now discussed under appropriate section headings. However, the teachings under each section are not necessarily limited to each particular section.

The carrier of the nicotine solution may be any suitable solvent such that the nicotine solution can be vaporised for use. **In** one aspect the solvent is selected from glycerol, propylene glycol and mixtures thereof. **In** one aspect the solvent is at least glycerol. **In** one aspect the solvent consists essentially of glycerol. **In** one aspect the solvent consists of glycerol. **In** one aspect the solvent is at least propylene glycol. **In** one aspect the solvent consists essentially of propylene glycol. **In** one aspect the solvent consists of propylene glycol. **In** one aspect the solvent is at least a mixture of propylene glycol and glycerol. **In** one aspect the solvent consists essentially of a mixture of propylene glycol and glycerol. **In** one aspect the solvent consists of a mixture of propylene glycol and glycerol.

The carrier of the nicotine solution may be present in any suitable amount. **In** one aspect the carrier is present in an amount of 1 to 98 wt% based on the solution. **In** one aspect the carrier is present in an amount of 5 to 98 wt% based on the solution. **In** one aspect the carrier is present in an amount of 10 to 98 wt% based on the solution. **In** one aspect the carrier is present in an amount of 20 to 98 wt% based on the solution. **In** one aspect the carrier is present in an amount of 30 to 98 wt% based on the solution. **In** one aspect the carrier is present in an amount of 40 to 98 wt% based on the solution. **In** one aspect the carrier is present in an amount of 50 to 98 wt% based on the solution. In one aspect the carrier is present in an amount of 60 to 98 wt% based on the solution. In one aspect the carrier is present in an amount of 70 to 98 wt% based on the solution. In one aspect the carrier is present in an amount of 80 to 98 wt% based on the solution. In one aspect the carrier is present in an amount of 90 to 98 wt% based on the solution. In one aspect the carrier is present in an amount of 1 to 90 wt% based on the solution. In one aspect the carrier is present in an amount of 5 to 90 wt% based on the solution. In one aspect the carrier is present in an amount of 10 to 90 wt% based on the solution. In one aspect the carrier is present in an amount of 20 to 90 wt% based on the solution. In one aspect the carrier is present in an amount of 30 to 90 wt% based on the solution. In one aspect the carrier is present in an amount of 40 to 90 wt% based on the solution. In one aspect the carrier is present in an amount of 50 to 90 wt% based on the solution. In one aspect the carrier is present in an amount of 60 to 90 wt% based on the solution. In one aspect the carrier is present in an amount of 70 to 90 wt% based on the solution. In one aspect the carrier is present in an amount of 80 to 90 wt% based on the solution.

The nicotine solution may also comprise flavouring components. In this case the carrier may preferably be propylene glycol. As used herein, the terms "flavour" and "flavourant" refer to materials which, where local regulations permit, may be used to create a desired taste or aroma in a product for adult consumers. They may include extracts (e.g. liquorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

In one aspect the nicotine solution further comprises water. The water may be present in any suitable amount. In one aspect water is present in an amount of 1 to 50 wt% based on the solution. In one aspect water is present in an amount of 5 to 50 wt% based on the solution. In one aspect water is present in an amount of 10 to 50 wt% based on the solution. In one aspect water is present in an amount of 20 to 50 wt% based on the solution. In one aspect water is present in an amount of 1 to 40 wt% based on the solution. In one aspect water is present in an amount of 5 to 40 wt% based on the solution. In one aspect water is present in an amount of 10 to 40 wt% based on the solution. In one aspect water is present in an amount of 20 to 40 wt% based on the solution. In one aspect water is present in an amount of 1 to 30 wt% based on the solution. In one aspect water is present in an amount of 5 to 30 wt% based on the solution. In one aspect water is present in an amount of 10 to 30 wt% based on the solution. In one aspect water is present in an amount of 20 to 30 wt% based on the solution.

In one aspect the combined amount of carrier and water in the nicotine solution is from 1 to 98 wt% based on the solution. In one aspect the combined amount of carrier and water in the nicotine solution is 5 to 98 wt% based on the solution. In one aspect the combined amount of carrier and water in the nicotine solution is 10 to 98 wt% based on the solution. In one aspect the combined amount of carrier and water in the nicotine solution is 20 to 98 wt% based on the solution. In one aspect the combined amount of carrier and water in the nicotine solution is 30 to 98 wt% based on the solution. In one aspect the combined amount of carrier and water in the nicotine solution is 40 to 98 wt% based on the solution. In one aspect the combined amount of carrier and water in the nicotine solution is 50 to 98 wt% based on the solution. In one aspect the combined amount of carrier and water in the nicotine solution is 60 to 98 wt% based on the solution. In one aspect the combined amount of carrier and water in the nicotine solution is 70 to 98 wt% based on the solution. In one aspect the combined amount of carrier and water in the nicotine solution is 80 to 98 wt% based on the solution. In one aspect the combined amount of carrier and water in the nicotine solution is 90 to 98 wt% based on the solution. In one aspect the combined amount of carrier and water in the nicotine solution is 1 to 90 wt% based on the solution. In one aspect the combined amount of carrier and water in the nicotine solution is 5 to 90 wt% based on the solution. In one aspect the combined amount of carrier and water in the nicotine solution is 10 to 90 wt% based on the solution. In one aspect the combined amount of carrier and water in the nicotine solution is 20 to 90 wt% based on the solution. In one aspect the combined amount of carrier and water in the nicotine solution is 30 to 90 wt% based on the solution. In one aspect the combined amount of carrier and water in the nicotine solution is 40 to 90 wt% based on the solution. In one aspect the combined amount of carrier and water in the nicotine solution is 50 to 90 wt% based on the solution. In one aspect the combined amount of carrier and water in the nicotine solution is 60 to 90 wt% based on the solution. In one aspect the combined amount of carrier and water in the nicotine solution is 70 to 90 wt% based on the solution. In one aspect the combined amount of carrier and water in the nicotine solution is 80 to 90 wt% based on the solution. In one aspect the combined amount of carrier and water in the nicotine solution is 90 to 90 wt% based on the solution.

In one aspect the nicotine solution may contain solvents which advantageously allow for the preparation of the formulation. In one aspect, the nicotine solution contains ethanol which improves the solubility of benzoic acid when incorporated into the formulation.

The components of the system may be present in the following amounts. The water may represent up to 30% w/w of the total solution. The carrier may represent up to 98% w/w of the total solution. The nicotine may represent from greater than 0% to 6% w/w of the total solution.

In the context of the present invention, reference to a nicotine solution comprising nicotine in both protonated form and in unprotonated form generally means that the amount of nicotine in unprotonated form is not minimal. For example, the amount of non-protonated nicotine is typically greater than 1% w/w.

The nicotine solution comprises nicotine in unprotonated form and nicotine in protonated form. In one aspect the nicotine solution comprises nicotine in unprotonated form and nicotine in monoprotonated form. Although it is envisaged that the solution will typically comprise nicotine in unprotonated form and nicotine in monoprotonated form, it may be that small amounts of diprotonated nicotine are present. In one aspect the nicotine solution comprises nicotine in unprotonated form, nicotine in monoprotonated form and nicotine in diprotonated form.

As discussed herein, we have found that by protonating a portion of the nicotine and only a portion of the nicotine the desirable characteristics are observed. In one aspect from 1 to 80 wt% of the nicotine present in the solution is in protonated form. In one aspect from 2 to 80 wt% of the nicotine present in the solution is in protonated form. In one aspect from 3 to 80 wt% of the nicotine present in the solution is in protonated form. In one aspect from 4 to 80 wt% of the nicotine present in the solution is in protonated form. In one aspect from 5 to 80 wt% of the nicotine present in the solution is in protonated form. In one aspect from 10 to 80 wt% of the nicotine present in the solution is in protonated form. In one aspect from 15 to 80 wt% of the nicotine present in the solution is in protonated form. In one aspect from 20 to 80 wt% of the nicotine present in the solution is in protonated form. In one aspect from 25 to 80 wt% of the nicotine present in the solution is in protonated form. In one aspect from 30 to 80 wt% of the nicotine present in the solution is in protonated form. In one aspect from 35 to 80 wt% of the nicotine present in the solution is in protonated form. In one aspect from 40 to 80 wt% of the nicotine present in the solution is in protonated form. In one aspect from 45 to 80 wt% of the nicotine present in the solution is in protonated form. In one aspect from 50 to 80 wt% of the nicotine present in the solution is in protonated form. **In** one aspect from 55 to 80 wt% of the nicotine present in the solution is in protonated form.

**In** one aspect from 5 to 80 wt% of the nicotine present in the solution is in protonated form. **In** one aspect from 5 to 75 wt% of the nicotine present in the solution is in protonated form. **In** one aspect from 5 to 70 wt% of the nicotine present in the solution is in protonated form. **In** one aspect from 5 to 65 wt% of the nicotine present in the solution is in protonated form. **In** one aspect from 5 to 60 wt% of the nicotine present in the solution is in protonated form. **In** one aspect from 5 to 55 wt% of the nicotine present in the solution is in protonated form. **In** one aspect from 5 to 50 wt% of the nicotine present in the solution is in protonated form. **In** one aspect from 5 to 45 wt% of the nicotine present in the solution is in protonated form. **In** one aspect from 5 to 40 wt% of the nicotine present in the solution is in protonated form. **In** one aspect from 5 to 35 wt% of the nicotine present in the solution is in protonated form. **In** one aspect from 5 to 30 wt% of the nicotine present in the solution is in protonated form. **In** one aspect from 5 to 25 wt% of the nicotine present in the solution is in protonated form. **In** one aspect from 5 to 20 wt% of the nicotine present in the solution is in protonated form. **In** one aspect from 5 to 15 wt% of the nicotine present in the solution is in protonated form. **In** one aspect from 5 to 10 wt% of the nicotine present in the solution is in protonated form.

The relevant amounts of nicotine which are present in the solution in protonated form are specified herein. These amounts may be readily calculated by one skilled in the art. Nicotine, 3-(1-methylpyrrolidin-2-yl) pyridine, is a diprotic base with pKa of 3.12 for the pyridine ring and 8.02 for the pyrrolidine ring It can exist in pH-dependent protonated (mono- and di-) and non-protonated (free base) forms which have different bioavailability.

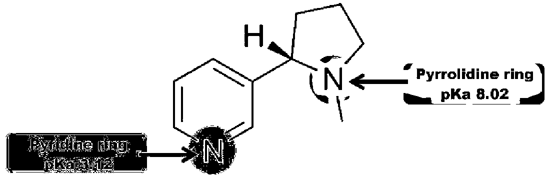

The distribution of protonated and non-protonated nicotine will vary at various pH increments.

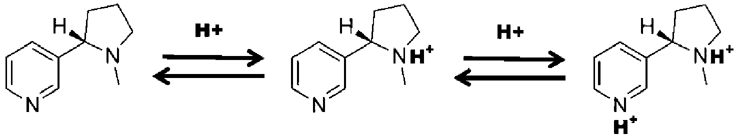

The fraction of non-protonated nicotine will be predominant at high pH levels whilst a decrease in the pH will see an increase of the fraction of protonated nicotine (mono- or di- depending on the pH). If the relative fraction of protonated nicotine and the total amount of nicotine in the sample are known, the absolute amount of protonated nicotine can be calculated.

The relative fraction of protonated nicotine in solution can be calculated by using the Henderson-Hasselbalch equation, which describes the pH as a derivation of the acid dissociation constant equation, and it is extensively employed in chemical and biological systems. Consider the following equilibrium:

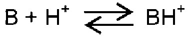

The Henderson-Hasselbalch equation for this equilibrium is: $pH = pKa + log \frac{\left[B\right]}{\left[BH+\right]}$

Where [B] is the amount of non-protonated nicotine (i.e. free base), [BH+] the amount of protonated nicotine (i.e. conjugate acid) and pKa is the reference pKa value for the pyrrolidine ring nitrogen of nicotine (pKa=8.02). The relative fraction of protonated nicotine can be derived from the alpha value of the non-protonated nicotine calculated from the Henderson-Hasselbalch equation as: $%protonated nicotine = 100 - \left\{\frac{\frac{\left[B\right]}{\left[BH+\right]}}{1 + \frac{\left[B\right]}{\left[BH+\right]}}∗100\right\}$

Determination of pKa values of nicotine solutions was carried out using the basic approach described in "Spectroscopic investigations into the acid-base properties of nicotine at different temperatures", Peter M. Clayton, Carl A. Vas, Tam T. T. Bui, Alex F. Drake and Kevin McAdam, .Anal. Methods, 2013,5, 81-88.

As discussed herein the nicotine solution comprises nicotine in unprotonated form and nicotine in protonated form. As will be understood by one skilled in the art, the protonated form of nicotine is prepared by reacting unprotonated nicotine with an acid. The acids are one or more suitable acids, wherein at least benzoic acid, levulinic acid or a mixture thereof is present; and wherein the total content of acid present in the solution is no greater than 0.6 mole equivalents based on the nicotine. As is clear, at least benzoic acid, levulinic acid or a mixture thereof must be present. However, one or more acids in addition to the benzoic acid and/or levulinic acid may also be present. The presence of acids in addition to benzoic acid and levulinic acid is not excluded nor is it required. Thus in a further aspect, the present invention provides a nicotine solution comprising
(i) a carrier;
(ii) nicotine in unprotonated form and in protonated form; and
(iii) a first acid, wherein the first acid is selected from benzoic acid, levulinic acid and mixtures thereof; and
(iv) an optional second acid, wherein the optional second acid, if present, is selected from acids other than benzoic acid, levulinic acid, and mixtures thereof; and
   wherein the total content of first acid and second acid present in the solution is no greater than 0.6 mole equivalents based on the nicotine.

The nicotine protonation may be provided in such a manner to achieve the desired degree of protonation of nicotine. In one aspect the optional second acid is an organic acid. In one aspect the optional second acid is a carboxylic acid. The carboxylic acid may be any suitable carboxylic acid. In one aspect the optional second acid is a mono-carboxylic acid.

In one aspect the optional second acid is selected from the group consisting of acetic acid, lactic acid, formic acid, citric acid, pyruvic acid, succinic acid, tartaric acid, oleic acid, sorbic acid, propionic acid, phenylacetic acid, and mixtures thereof.

In one aspect of the present invention, at least benzoic acid is present in the solution. In one aspect of the present invention, at least levulinic acid is present in the solution. In one aspect of the present invention, benzoic acid and levulinic acid are present in the solution.

As discussed herein the presence of acids in addition to benzoic acid and levulinic acid is not required. In one aspect, the presence of acids in addition to benzoic acid and levulinic acid is excluded. Thus in one aspect the nicotine solution contains acids selected from the group consisting of benzoic acid, levulinic acid and mixtures thereof. Thus in one aspect the present invention provides a nicotine solution comprising (i) a solvent; (ii) nicotine in unprotonated form and in protonated form; and (iii) acid selected from the group consisting of benzoic acid, levulinic acid and mixtures thereof; and wherein the total content of acid present in the solution is no greater than 0.6 mole equivalents based on the nicotine.

In one aspect benzoic acid is the only acid present. In one aspect the nicotine solution contains acid selected from the group consisting of benzoic acid.

In one aspect levulinic acid is the only acid present. In one aspect the nicotine solution contains acid selected from the group consisting of levulinic acid.

In one aspect benzoic acid and levulinic acid are the only acids present. In one aspect the nicotine solution contains acids selected from the group consisting of mixtures of benzoic acid and levulinic acid.

In one aspect the amount of levulinic acid present in the solution is less than 0.1 mole equivalents based on the nicotine. In one aspect the amount of levulinic acid present in the solution is no greater than 0.05 mole equivalents based on the nicotine. In one aspect the amount of levulinic acid present in the solution is no greater than 0.02 mole equivalents based on the nicotine. In one aspect the amount of levulinic acid present in the solution is no greater than 0.01 mole equivalents based on the nicotine. In one aspect the amount of levulinic acid present in the solution is no greater than 0.005 mole equivalents based on the nicotine. In one aspect the amount of levulinic acid present in the solution is no greater than 0.001 mole equivalents based on the nicotine. In one aspect the solution contain no levulinic acid.

The acids benzoic acid and levulinic acid are advantageous since we have found that on heating solutions containing benzoic acid and/or levulinic acid in an electronic vapour provision system the level of acid transfer to the aerosol is greater, with less production of degradation products compared to many other acids. Thus, we have found that the aerosol transfer for these acids is more efficient.

We have also found that benzoic acid provides a particularly desirable taste when the vaporised solution is inhaled. Thus in contrast to acids such as lactic acid, acetic acid and succinic acid, benzoic acid provides both good flavour and/or improved aerosol transfer efficiency. Thus, as disclosed herein in one aspect of the present invention, at least benzoic acid is present in the solution. Indeed when benzoic acid is present, the total limit on acid content described herein need not be observed. Thus in a further broad aspect the present invention provides a nicotine solution comprising
(i) a carrier;
(ii) nicotine in protonated form and in unprotonated form; and
(iii) benzoic acid.

In one aspect when benzoic acid is present, the nicotine solution contains no greater than 0.1 mole equivalents based on the nicotine of each of lactic acid, acetic acid and succinic acid. In one aspect the nicotine solution contains no greater than 0.01 mole equivalents based on the nicotine of each of lactic acid, acetic acid and succinic acid. Each of the preferred aspects of the present invention are equally applicable to this broad aspect of the invention.

The total content of acid present in the solution is no greater than 0.6 mole equivalents based on the nicotine. In one aspect the total content of acid present in the solution is no greater than 0.55 mole equivalents based on the nicotine. In one aspect the total content of acid present in the solution is no greater than 0.5 mole equivalents based on the nicotine. In one aspect the total content of acid present in the solution is no greater than 0.45 mole equivalents based on the nicotine. In one aspect the total content of acid present in the solution is no greater than 0.4 mole equivalents based on the nicotine. In one aspect the total content of acid present in the solution is no greater than 0.35 mole equivalents based on the nicotine. In one aspect the total content of acid present in the solution is no greater than 0.3 mole equivalents based on the nicotine.

In one aspect the combined amount of benzoic acid and levulinic acid present in the solution is no greater than 0.6 mole equivalents based on the nicotine. In one aspect the combined amount of benzoic acid and levulinic acid present in the solution is no greater than 0.55 mole equivalents based on the nicotine. In one aspect the combined amount of benzoic acid and levulinic acid present in the solution is no greater than 0.5 mole equivalents based on the nicotine. In one aspect the combined amount of benzoic acid and levulinic acid present in the solution is no greater than 0.45 mole equivalents based on the nicotine. In one aspect the combined amount of benzoic acid and levulinic acid present in the solution is no greater than 0.4 mole equivalents based on the nicotine. In one aspect the combined amount of benzoic acid and levulinic acid present in the solution is no greater than 0.35 mole equivalents based on the nicotine. In one aspect the combined amount of benzoic acid and levulinic acid present in the solution is no greater than 0.3 mole equivalents based on the nicotine.

The amount of benzoic acid present in the solution is no greater than 0.6 mole equivalents based on the nicotine. In one aspect the amount of benzoic acid present in the solution is no greater than 0.55 mole equivalents based on the nicotine. In one aspect the amount of benzoic acid present in the solution is no greater than 0.5 mole equivalents based on the nicotine. In one aspect the amount of benzoic acid present in the solution is no greater than 0.45 mole equivalents based on the nicotine. In one aspect the amount of benzoic acid present in the solution is no greater than 0.4 mole equivalents based on the nicotine. In one aspect the amount of benzoic acid present in the solution is no greater than 0.35 mole equivalents based on the nicotine. In one aspect the amount of benzoic acid present in the solution is no greater than 0.3 mole equivalents based on the nicotine. In each of these aspects, preferably benzoic acid is the only acid present and the nicotine solution contains acid selected from the group consisting of benzoic acid.

In one aspect the total content of acid present in the solution is no less than 0.01 mole equivalents based on the nicotine. In one aspect the total content of acid present in the solution is no less than 0.05 mole equivalents based on the nicotine. In one aspect the total content of acid present in the solution is no less than 0.1 mole equivalents based on the nicotine. In one aspect the total content of acid present in the solution is no less than 0.15 mole equivalents based on the nicotine. In one aspect the total content of acid present in the solution is no less than 0.2 mole equivalents based on the nicotine. In one aspect the total content of acid present in the solution is no less than 0.25 mole equivalents based on the nicotine. In one aspect the total content of acid present in the solution is no less than 0.3 mole equivalents based on the nicotine. In one aspect the total content of acid present in the solution is no less than 0.35 mole equivalents based on the nicotine. In one aspect the total content of acid present in the solution is no less than 0.4 mole equivalents based on the nicotine.

In one aspect the combined amount of benzoic acid and levulinic acid present in the solution is no less than 0.01 mole equivalents based on the nicotine. In one aspect the combined amount of benzoic acid and levulinic acid present in the solution is no less than 0.05 mole equivalents based on the nicotine. In one aspect the combined amount of benzoic acid and levulinic acid present in the solution is no less than 0.1 mole equivalents based on the nicotine. In one aspect the combined amount of benzoic acid and levulinic acid present in the solution is no less than 0.15 mole equivalents based on the nicotine. In one aspect the combined amount of benzoic acid and levulinic acid present in the solution is no less than 0.2 mole equivalents based on the nicotine. In one aspect the combined amount of benzoic acid and levulinic acid present in the solution is no less than 0.25 mole equivalents based on the nicotine. In one aspect the combined amount of benzoic acid and levulinic acid present in the solution is no less than 0.3 mole equivalents based on the nicotine. In one aspect the combined amount of benzoic acid and levulinic acid present in the solution is no less than 0.35 mole equivalents based on the nicotine. In one aspect the combined amount of benzoic acid and levulinic acid present in the solution is no less than 0.4 mole equivalents based on the nicotine.

In one aspect the amount of benzoic acid present in the solution is no less than 0.01 mole equivalents based on the nicotine. In one aspect the amount of benzoic acid present in the solution is no less than 0.05 mole equivalents based on the nicotine. In one aspect the amount of benzoic acid present in the solution is no less than 0.1 mole equivalents based on the nicotine. In one aspect the amount of benzoic acid present in the solution is no less than 0.15 mole equivalents based on the nicotine. In one aspect the amount of benzoic acid present in the solution is no less than 0.2 mole equivalents based on the nicotine. In one aspect the amount of benzoic acid present in the solution is no less than 0.25 mole equivalents based on the nicotine. In one aspect the amount of benzoic acid present in the solution is no less than 0.3 mole equivalents based on the nicotine. In one aspect the amount of benzoic acid present in the solution is no less than 0.35 mole equivalents based on the nicotine. In one aspect the amount of benzoic acid present in the solution is no less than 0.4 mole equivalents based on the nicotine.

In one aspect the total content of acid present in the solution is from 0.1 to 0.6 mole equivalents based on the nicotine. In one aspect the total content of acid present in the solution is from 0.1 to 0.5 mole equivalents based on the nicotine. In one aspect the total content of acid present in the solution is from 0.2 to 0.6 mole equivalents based on the nicotine. In one aspect the total content of acid present in the solution is from 0.1 to 0.4 mole equivalents based on the nicotine. In one aspect the total content of acid present in the solution is from 0.3 to 0.6 mole equivalents based on the nicotine. In one aspect the total content of acid present in the solution is from 0.2 to 0.5 mole equivalents based on the nicotine. In one aspect the total content of acid present in the solution is from 0.3 to 0.5 mole equivalents based on the nicotine. In one aspect the total content of acid present in the solution is from 0.2 to 0.4 mole equivalents based on the nicotine.

In one aspect the combined amount of benzoic acid and levulinic acid present in the solution is from 0.1 to 0.6 mole equivalents based on the nicotine. In one aspect the combined amount of benzoic acid and levulinic acid present in the solution is from 0.1 to 0.5 mole equivalents based on the nicotine. In one aspect the combined amount of benzoic acid and levulinic acid present in the solution is from 0.2 to 0.6 mole equivalents based on the nicotine. In one aspect the combined amount of benzoic acid and levulinic acid present in the solution is from 0.1 to 0.4 mole equivalents based on the nicotine. In one aspect the combined amount of benzoic acid and levulinic acid present in the solution is from 0.3 to 0.6 mole equivalents based on the nicotine. In one aspect the combined amount of benzoic acid and levulinic acid present in the solution is from 0.2 to 0.5 mole equivalents based on the nicotine. In one aspect the combined amount of benzoic acid and levulinic acid present in the solution is from 0.3 to 0.5 mole equivalents based on the nicotine. In one aspect the combined amount of benzoic acid and levulinic acid present in the solution is from 0.2 to 0.4 mole equivalents based on the nicotine.

In one aspect the amount of benzoic acid present in the solution is from 0.1 to 0.6 mole equivalents based on the nicotine. In one aspect the amount of benzoic acid present in the solution is from 0.1 to 0.5 mole equivalents based on the nicotine. In one aspect the amount of benzoic acid present in the solution is from 0.2 to 0.6 mole equivalents based on the nicotine. In one aspect the amount of benzoic acid present in the solution is from 0.1 to 0.4 mole equivalents based on the nicotine. In one aspect the amount of benzoic acid present in the solution is from 0.3 to 0.6 mole equivalents based on the nicotine. In one aspect the amount of benzoic acid present in the solution is from 0.2 to 0.5 mole equivalents based on the nicotine. In one aspect the amount of benzoic acid present in the solution is from 0.3 to 0.5 mole equivalents based on the nicotine. In one aspect the amount of benzoic acid present in the solution is from 0.2 to 0.4 mole equivalents based on the nicotine. In each of these aspects, preferably benzoic acid is the only acid present and the nicotine solution contains acid selected from the group consisting of benzoic acid.

As discussed herein we have found that at levels of acid addition required by the present invention, namely wherein the total content of acid present in the solution is no greater than 0.6 mole equivalents based on the nicotine, may be used across a broad range of nicotine content solutions. Nicotine solutions may be provided having desirable properties of flavour, impact, irritation, smoothness and/or nicotine reward for the user both when the nicotine content is relatively low, such as 1.9 wt% or 1.8wt% nicotine or less and when the nicotine content is relatively high, such as greater than 1.9 wt% or 1.8wt% nicotine. Thus in one aspect the nicotine solution comprises nicotine in an amount of no greater than 1.9 wt% or 1.8 wt% based on the total weight of the solution. Thus in one aspect the nicotine solution comprises nicotine in an amount of greater than 1.9 wt% or 1.8 wt% based on the total weight of the solution.

Nicotine may be provided at any suitable amount depending on the desired dosage when inhaled by the user. In one aspect nicotine is present in an amount of no greater than 6 wt% based on the total weight of the solution. In one aspect nicotine is present in an amount of from 0.4 to 6 wt% based on the total weight of the solution. In one aspect nicotine is present in an amount of from 0.8 to 6 wt% based on the total weight of the solution. In one aspect nicotine is present in an amount of from 1 to 6 wt% based on the total weight of the solution. In one aspect nicotine is present in an amount of from 1.8 to 6 wt% based on the total weight of the solution. In one aspect nicotine is present in an amount of from 0.4 to 5 wt% based on the total weight of the solution. In one aspect nicotine is present in an amount of from 0.8 to 5 wt% based on the total weight of the solution. In one aspect nicotine is present in an amount of from 1 to 5 wt% based on the total weight of the solution. In one aspect nicotine is present in an amount of from 1.8 to 5 wt% based on the total weight of the solution. In one aspect nicotine is present in an amount of no greater than 4 wt% based on the total weight of the solution. In one aspect nicotine is present in an amount of from 0.4 to 4 wt% based on the total weight of the solution. In one aspect nicotine is present in an amount of from 0.8 to 4 wt% based on the total weight of the solution. In one aspect nicotine is present in an amount of from 1 to 4 wt% based on the total weight of the solution. In one aspect nicotine is present in an amount of from 1.8 to 4 wt% based on the total weight of the solution. In one aspect nicotine is present in an amount of no greater than 3 wt% based on the total weight of the solution. In one aspect nicotine is present in an amount of from 0.4 to 3 wt% based on the total weight of the solution. In one aspect nicotine is present in an amount of from 0.8 to 3 wt% based on the total weight of the solution. In one aspect nicotine is present in an amount of from 1 to 3 wt% based on the total weight of the solution. In one aspect nicotine is present in an amount of from 1.8 to 3 wt% based on the total weight of the solution. In one aspect nicotine is present in an amount of no greater than 1.9 wt% based on the total weight of the solution. In one aspect nicotine is present in an amount of no greater than 1.8 wt% based on the total weight of the solution. In one aspect nicotine is present in an amount of from 0.4 to 1.9 wt% based on the total weight of the solution. In one aspect nicotine is present in an amount of from 0.4 to 1.8 wt% based on the total weight of the solution. In one aspect nicotine is present in an amount of from 0.5 to 1.9 wt% based on the total weight of the solution. In one aspect nicotine is present in an amount of from 0.5 to 1.8 wt% based on the total weight of the solution In one aspect nicotine is present in an amount of from 0.8 to 1.9 wt% based on the total weight of the solution.. In one aspect nicotine is present in an amount of from 0.8 to 1.8 wt% based on the total weight of the solution. In one aspect nicotine is present in an amount of from 1 to 1.9 wt% based on the total weight of the solution. In one aspect nicotine is present in an amount of from 1 to 1.8 wt% based on the total weight of the solution. In one aspect nicotine is present in an amount of less than 1.9 wt% based on the total weight of the solution. In one aspect nicotine is present in an amount of less than 1.8 wt% based on the total weight of the solution. In one aspect nicotine is present in an amount of from 0.4 to less than 1.9 wt% based on the total weight of the solution. In one aspect nicotine is present in an amount of from 0.4 to less than 1.8 wt% based on the total weight of the solution. In one aspect nicotine is present in an amount of from 0.5 to less than 1.9 wt% based on the total weight of the solution. In one aspect nicotine is present in an amount of from 0.5 to less than 1.8 wt% based on the total weight of the solution. In one aspect nicotine is present in an amount of from 0.8 to less than 1.9 wt% based on the total weight of the solution. In one aspect nicotine is present in an amount of from 0.8 to less than 1.8 wt% based on the total weight of the solution. In one aspect nicotine is present in an amount of from 1 to less than 1.9 wt% based on the total weight of the solution. In one aspect nicotine is present in an amount of from 1 to less than 1.8 wt% based on the total weight of the solution.

In one aspect, when levulinic acid is present, nicotine is present in an amount of no greater than 1.9 wt% based on the total weight of the solution. In one aspect, when levulinic acid is present, nicotine is present in an amount of from 0.4 to 1.9 wt% based on the total weight of the solution. In one aspect, when levulinic acid is present, nicotine is present in an amount of from 0.5 to 1.9 wt% based on the total weight of the solution. In one aspect, when levulinic acid is present, nicotine is present in an amount of from 0.8 to 1.9 wt% based on the total weight of the solution. In one aspect, when levulinic acid is present, nicotine is present in an amount of from 1 to 1.9 wt% based on the total weight of the solution. In one aspect, when levulinic acid is present, nicotine is present in an amount of less than 1.9 wt% based on the total weight of the solution. In one aspect, when levulinic acid is present, nicotine is present in an amount of from 0.4 to less than 1.9 wt% based on the total weight of the solution. In one aspect, when levulinic acid is present, nicotine is present in an amount of from 0.5 to less than 1.9 wt% based on the total weight of the solution. In one aspect, when levulinic acid is present, nicotine is present in an amount of from 0.8 to less than 1.9 wt% based on the total weight of the solution. In one aspect, when levulinic acid is present, nicotine is present in an amount of from 1 to less than 1.9 wt% based on the total weight of the solution.

In one aspect, when levulinic acid is present, nicotine is present in an amount of no greater than 1.8 wt% based on the total weight of the solution. In one aspect, when levulinic acid is present, nicotine is present in an amount of from 0.4 to 1.8 wt% based on the total weight of the solution. In one aspect, when levulinic acid is present, nicotine is present in an amount of from 0.5 to 1.8 wt% based on the total weight of the solution. In one aspect, when levulinic acid is present, nicotine is present in an amount of from 0.8 to 1.8 wt% based on the total weight of the solution. In one aspect, when levulinic acid is present, nicotine is present in an amount of from 1 to 1.8 wt% based on the total weight of the solution. In one aspect, when levulinic acid is present, nicotine is present in an amount of less than 1.8 wt% based on the total weight of the solution. In one aspect, when levulinic acid is present, nicotine is present in an amount of from 0.4 to less than 1.8 wt% based on the total weight of the solution. In one aspect, when levulinic acid is present, nicotine is present in an amount of from 0.5 to less than 1.8 wt% based on the total weight of the solution. In one aspect, when levulinic acid is present, nicotine is present in an amount of from 0.8 to less than 1.8 wt% based on the total weight of the solution. In one aspect, when levulinic acid is present, nicotine is present in an amount of from 1 to less than 1.8 wt% based on the total weight of the solution.

In one aspect nicotine is present in an amount of less than 1.8 wt% and the acids present are only benzoic acid, levulinic acid or mixtures thereof. Thus in one aspect the present invention provides a nicotine solution comprising (i) a carrier; (ii) nicotine in unprotonated form and in protonated form, wherein nicotine is present in an amount of less than 1.8 wt% based on the total weight of the solution; and (iii) acid selected from the group consisting of benzoic acid, levulinic acid and mixtures thereof; and wherein the total content of acid present in the solution is no greater than 0.6 mole equivalents based on the nicotine. In this aspect the combined amount of benzoic acid and levulinic acid present in the solution may be from 0.1 to 0.6 mole equivalents based on the nicotine, such as from 0.1 to 0.5 mole equivalents based on the nicotine, such as from 0.2 to 0.6 mole equivalents based on the nicotine, such as from 0.1 to 0.4 mole equivalents based on the nicotine, such as from 0.3 to 0.6 mole equivalents based on the nicotine, such as from 0.2 to 0.5 mole equivalents based on the nicotine, such as from 0.3 to 0.5 mole equivalents based on the nicotine, such as from 0.2 to 0.4 mole equivalents based on the nicotine.

In one aspect nicotine is present in an amount of less than 1.9 wt% and the acids present are only benzoic acid. Thus in one aspect the present invention provides a nicotine solution comprising (i) a carrier; (ii) nicotine in unprotonated form and in protonated form, wherein nicotine is present in an amount of less than 1.9 wt% based on the total weight of the solution; and (iii) acid selected from the group consisting of benzoic acid; and wherein the total content of acid present in the solution is no greater than 0.6 mole equivalents based on the nicotine. In this aspect the amount of benzoic acid present in the solution may be from 0.1 to 0.6 mole equivalents based on the nicotine, such as from 0.1 to 0.5 mole equivalents based on the nicotine, such as from 0.2 to 0.6 mole equivalents based on the nicotine, such as from 0.1 to 0.4 mole equivalents based on the nicotine, such as from 0.3 to 0.6 mole equivalents based on the nicotine, such as from 0.2 to 0.5 mole equivalents based on the nicotine, such as from 0.3 to 0.5 mole equivalents based on the nicotine, such as from 0.2 to 0.4 mole equivalents based on the nicotine.

In one aspect nicotine is present in an amount of less than 1.8 wt% and the acids present are only benzoic acid. Thus in one aspect the present invention provides a nicotine solution comprising (i) a carrier; (ii) nicotine in unprotonated form and in protonated form, wherein nicotine is present in an amount of less than 1.8 wt% based on the total weight of the solution; and (iii) acid selected from the group consisting of benzoic acid; and wherein the total content of acid present in the solution is no greater than 0.6 mole equivalents based on the nicotine. In this aspect the amount of benzoic acid present in the solution may be from 0.1 to 0.6 mole equivalents based on the nicotine, such as from 0.1 to 0.5 mole equivalents based on the nicotine, such as from 0.2 to 0.6 mole equivalents based on the nicotine, such as from 0.1 to 0.4 mole equivalents based on the nicotine, such as from 0.3 to 0.6 mole equivalents based on the nicotine, such as from 0.2 to 0.5 mole equivalents based on the nicotine, such as from 0.3 to 0.5 mole equivalents based on the nicotine, such as from 0.2 to 0.4 mole equivalents based on the nicotine.

As will be understood by one skilled in the art, the present invention requires that the nicotine be partially protonated prior to vaporisation. This protonation may occur at any time before vaporisation. In one aspect the nicotine is partially protonated very shortly prior to vaporisation. For example the nicotine may be partially protonated as part of the process to provide vaporisation. Thus it is envisaged that an 'inline' process may be provided in which nicotine in unprotonated form is contacted with the desired acid and the partially protonated nicotine solution which is formed is then vaporised. It is also envisaged that the end user may be provided with the necessary acid and combine this with purchased nicotine in unprotonated form. The then partially protonated nicotine solution may then be used in an electronic vapour provision system in place of unprotonated nicotine. Thus in a further aspect there is provided a kit for a nicotine solution of the invention, the kit comprising (a) a nicotine solution comprising a carrier and nicotine in unprotonated form; and (b) one or more acids, wherein at least benzoic acid, levulinic acid or a mixture thereof is present; in separate packages or containers; with instructions for admixture and/or contacting and/or use to provide a partially protonated nicotine solution in which the total content of acid present in the solution is no greater than 0.6 mole equivalents based on the nicotine. In a further aspect there is also provided a process for improving the sensory properties of a vaporised nicotine solution, the process comprising the steps of
(a) providing a nicotine solution comprising
   (i) a carrier;
   (ii) nicotine in unprotonated form; and
(b) providing an acid solution comprising one or more acids, wherein at least benzoic acid, levulinic acid or a mixture thereof is present; and
(c) vaporising the nicotine solution and the acid solution
(d) combining the vaporised nicotine solution and the vaporised acid solution, such that the acid is present in an amount of no greater than 0.6 mole equivalents based on the nicotine.

The solution may be contained or delivered by any means. In one aspect the present invention provides a contained nicotine solution comprising (a) a container; and (b) a nicotine solution, comprising (i) a carrier; (ii) nicotine in unprotonated form and in protonated form; and (iii) one or more acids, wherein at least benzoic acid, levulinic acid or a mixture thereof is present; and wherein the total content of acid present in the solution is no greater than 0.6 mole equivalents based on the nicotine. The container may be any suitable container, for example to allow for the storage or delivery of the solution. In one aspect the container is configured for engagement with an electronic vapour provision system. The container may be configured to become fluidly in communication with an electronic vapour provision system so that solution may be delivered to the electronic vapour provision system. As described above, the present disclosure relates to container which may be used in an electronic vapour provision system, such as an e-cigarette. Throughout the following description the term "e-cigarette" is used; however, this term may be used interchangeably with electronic vapour provision system.

As discussed herein, the container of the present invention is typically provided for the delivery of nicotine solution to or within an e-cigarette. The nicotine solution may be held within an e-cigarette or may be sold as a separate container for subsequent use with or in an e-cigarette. As understood by one skilled in the art, e-cigarettes may contain a unit known as a detachable cartomiser which typically comprises a reservoir of nicotine solution, a wick material and a heating element for vaporising the nicotine. In some e-cigarettes, the cartomiser is part of a single-piece device and is not detachable. In one aspect the container is a cartomiser or is part of a cartomiser. In one aspect the container is not a cartomiser or part of a cartomiser and is a container, such as a tank, which may be used to deliver nicotine solution to or within an e-cigarette.

In one aspect the container is part of an e-cigarette. Therefore in a further aspect the present invention provides an electronic vapour provision system comprising:
a vaporiser for vaporising liquid for inhalation by a user of the electronic vapour provision system;
a power supply comprising a cell or battery for supplying power to the vaporiser a nicotine solution, comprising
   (i) a carrier;
   (ii) nicotine in unprotonated form and in protonated form; and
   (iii) one or more acids, wherein at least benzoic acid, levulinic acid or a mixture thereof is present; and
      wherein the total content of acid present in the solution is no greater than 0.6 mole equivalents based on the nicotine.

In addition to the solution of the present invention and to systems such as containers and electronic vapour provision systems containing the same, the present invention provides a process for improving the sensory properties of a vaporised nicotine solution. The process comprising the steps of (a) providing a nicotine solution comprising
(i) a carrier; (ii) nicotine in unprotonated form and in protonated form; and (iii) one or more acids, wherein at least benzoic acid, levulinic acid or a mixture thereof is present; and wherein the total content of acid present in the solution is no greater than 0.6 mole equivalents based on the nicotine; (b) vaporising the nicotine solution

Reference to an improvement in the sensory properties of a vaporised nicotine solution refer may include an improvement in the smoothness of the vaporised nicotine solution as perceived by a user.

The process of the present invention may comprises additional steps either before the steps listed, after the steps listed or between one or more of the steps listed.

In addition to the solution of the present invention and to systems such as containers and electronic vapour provision systems containing the same, the present invention provides use of one or more acids for improving sensory properties of a vaporised nicotine solution. In the use the nicotine solution comprises (i) a carrier; (ii) nicotine in unprotonated form and in protonated form; and (iii) one or more acids, wherein at least benzoic acid, levulinic acid or a mixture thereof is present; and wherein the total content of acid present in the solution is no greater than 0.6 mole equivalents based on the nicotine.

### BRIEF DESCRIPTION OF THE DRAWINGS

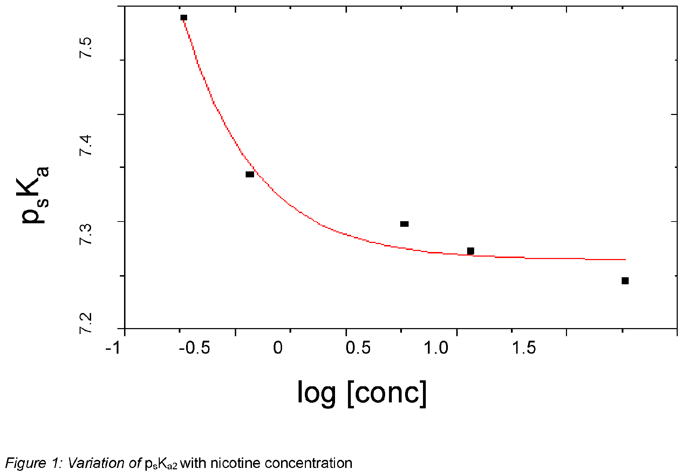

The present invention will now be described in further detail by way of example only with reference to the accompanying figure in which:-
Figure 1 shows a graph illustrating variation of pₛKₐ₂ with nicotine concentration

The invention will now be described with reference to the following non-limiting example.

### Examples

### Determination of pKa Values

The determination of pKa values of nicotine in glycerol/water systems was carried out using the basic approach described in "Spectroscopic investigations into the acid-base properties of nicotine at different temperatures", Peter M. Clayton, Carl A. Vas, Tam T. T. Bui, Alex F. Drake and Kevin McAdam, ·Anal. Methods, 2013,5, 81-88, and summarised below. Because the system is predominately non-aqueous the parameter pₛKₐ₂ was measured, where subscript s refers to the solvent composition in this largely non-aqueous system, and subscript 2 refers to the pKₐ value of the pyrrolidyl nitrogen.

Further information on the determination of pKa values of nicotine is provided in "Use of chiroptical spectroscopy to determine the ionisation status of (S)-nicotine in e-cigarette formulations and snus", Clayton et al, ST 49, CORESTA Congress, Québec City, Canada, 12-16 October 2014 (available at http://www.bat-science.com/groupms/sites/BAT_9GVJXS.nsf/vwPagesWebLive/DO9PVC3G/$FILE/CORES TA_PC_2014.pdf)

A range of glycerol/water/nicotine solutions were prepared, with the water concentration fixed at 9%, the nicotine concentration varying from 30µg/ml to 3mg/ml; and the glycerol content comprising the remainder of the solutions.

Simultaneous UV & CD spectra of glycerol/s-nicotine/water solutions were measured on the Applied Photophysics Ltd (Leatherhead, UK) Chiracsan Plus spectrometer. The UV absorbance & CD spectra were measured between 300-200 nm region, with various pathlengths depending upon the nicotine concentration of the solution - 10mm, 5mm, 2mm, 1mm, 0.5mm, 0.1mm and 0.01mm pathlengths. The instrument was flushed continuously with pure evaporated nitrogen throughout the measurements. Throughout measurements spectra were recorded with a 0.5 nm step size, a 1s measurement time-per-point and a spectral bandwidth of 2 nm. Where possible, all CD spectra were smoothed with a window factor of 4 using the Savitzky-Golay method for better presentation.

Solutions of S-Nicotine in glycerol/water were pH titrated at 23°C. The pH of these solutions was raised towards alkaline by adding small aliquots of NaOH (~pH10) and then lowered to pH2 by adding small aliquots of HCl. A series of 0.1M, 0.5M, 1M, 5M and 10M of HCl and NaOH solutions were used during the pH titration. pHs were measured at 23°C using a Corning pH105 pH meter with a RMS pH electrode. The pₛKₐ₂ values changed systematically with nicotine concentration (Figure 1) and therefore values for pₛKₐ₂ were calculated at each nicotine concentration level (Table 1)*. Due to the viscosity of the solutions, and the optical density in the CD spectra of the high nicotine concentration solutions, very small path-length cells were required for nicotine concentrations above 3mg*/*ml.* S*atisfactory sample preparation and spectroscopy could not be achieved with the necessary small cells at these concentrations, and therefore the* pₛKₐ₂ *at higher concentrations were calculated from a regression fit to* *Figure 1**.*

**Table 1: pₛKₐ₂ values measured at various nicotine concentrations in a 9% water, nicotine/glycerol system.**

| **p_{S}Kₐ₂** | **conc** (g/L) | **conc** (mM) | **log₁₀ [conc]** |
|---|---|---|---|
| 7.49 | 0.03 | 0.185 | -0.732 |
| 7.34 | 0.06 | 0.370 | -0.431 |
| 7.30 | 0.3 | 1.85 | 0.268 |
| 7.27 | 0.6 | 3.70 | 0.569 |
| 7.25 | 3 | 18.53 | 1.268 |

Curve fitting, using the equation y= 0.0233e^{(-(log10[nicotine])/0.325)} + 7.26 provided a pₛKₐ₂ value of 7.26 at 30 mg/ml nicotine concentration. Use of this pₛKₐ₂ value with the Henderson-Hasselbalch equation allows calculation of the degree of nicotine protonation at any pH value.

### Example 1

A series of tests were conducted using Vype E-pen electronic cigarettes. The "unprotonated nicotine control" devices were loaded with solution containing 1.86% (w/w) nicotine, 25% propylene glycol containing tobacco flavour "A", 25% water and 48.1% glycerol. A pH of 8.7 was measured for this solution, indicating 4% protonation of nicotine.

A similar set of devices were prepared wherein 0.55% w/w (0.4Meq to nicotine) benzoic acid was added to the formulation, with the glycerol content was commensurately reduced to 47.6% (w/w). A pH of 7.4 was measured for this solution, indicating 43% protonation of nicotine.

A third set of devices were prepared wherein 0.25% w/w (0.2Meq to nicotine) benzoic acid was added to the formulation, with the glycerol content was commensurately adjusted to 47.9% (w/w). A pH of 7.8 was measured for this solution, indicating 24% protonation of nicotine.

One each of these e-cigarettes was presented to 15 panellists comprising e-cigarette users, and the panellists were asked to puff on the e-cigarettes in a sequential monadic fashion for 10 puffs on each device. They were asked to identify the preferred e-cigarette from the three offered to them.

7 panellists preferred the unprotonated control e-cigarette, and 8 people preferred the acidified samples - 4 preferred the 0.2Meq device and 4 preferred the 0.4Meq device.

### Example 2

A series of tests were conducted using Vype E-pen electronic cigarettes. The "unprotonated nicotine control" devices were loaded with solution containing 1.86% (w/w) nicotine, 35.3% propylene glycol containing mint flavour, 25% water and 37.9 glycerol. This solution had a pH of 9.7 indicating <1% nicotine protonation.

A similar set of devices were prepared wherein 0.55% w/w (0.4Meq to nicotine) benzoic acid was added to the formulation, with the glycerol content was commensurately reduced to 37.3% (w/w). This solution had a pH of 7.4 indicating nicotine protonation of 43%.

A third set of devices were prepared wherein 0.25% w/w (0.2Meq to nicotine) benzoic acid was added to the formulation, with the glycerol content was commensurately adjusted to 37.6% (w/w). This solution had a pH of 7.8 indicating nicotine protonation of 22%.

One each of these e-cigarettes was presented to 15 panellists comprising e-cigarette users, and the panellists were asked to puff on the e-cigarettes in a sequential monadic fashion for 10 puffs on each device. They were asked to identify the preferred e-cigarette from the three offered to them.

4 panellists preferred the unprotonated control e-cigarette, and 11 people preferred the acidified samples - 2 preferred the 0.2Meq device and 9 preferred the 0.4Meq device.

### Example 3

A series of tests were conducted using Vype E-pen electronic cigarettes. The "unprotonated nicotine control" devices were loaded with solution containing 1.86% (w/w) nicotine, 25% propylene glycol containing a cherry flavour, 25% water and 48.1% glycerol. This solution had a pH of 8.4 indicating nicotine protonation at a level of 7%.

A similar set of devices were prepared wherein 0.55% w/w (0.4Meq to nicotine) benzoic acid was added to the formulation, with the glycerol content was commensurately reduced to 47.6% (w/w). This solution had a pH of 7.4 indicating nicotine protonation at a level of 43%.

A third set of devices were prepared wherein 0.25% w/w (0.2Meq to nicotine) benzoic acid was added to the formulation, with the glycerol content was commensurately adjusted to 47.9% (w/w). This solution had a pH of 7.8 indicating nicotine protonation at a level of 24%

One each of these e-cigarettes was presented to 15 panellists comprising e-cigarette users, and the panellists were asked to puff on the e-cigarettes in a sequential monadic fashion for 10 puffs on each device. They were asked to identify the preferred e-cigarette from the three offered to them.
3 panellists preferred the unprotonated control e-cigarette, and 12 people preferred the acidified samples - 8 preferred the 0.2Meq device and 4 preferred the 0.4Meq device.

### Example 4

A series of tests were conducted using Vype E-pen electronic cigarettes. The "unprotonated nicotine control" devices were loaded with solution containing 1.86% (w/w) nicotine, 25% propylene glycol containing tobacco flavour "A", 25% water and 48.1% glycerol. This solution had a pH of 8.6 indicating nicotine protonation at a level of 4%.

A similar set of devices were prepared wherein 0.41% w/w (0.3Meq to nicotine) benzoic acid was added to the formulation, with the glycerol content was commensurately reduced to 47.7% (w/w). This solution had a pH of 7.7 indicating nicotine protonation at a level of 26%. A third set of devices were prepared wherein 0.39% w/w (0.3Meq to nicotine) levulinic acid was added to the formulation, with the glycerol content was commensurately adjusted to 47.8% (w/w). This solution had a pH of 7.26 indicating nicotine protonation at a level of 50 %.

One each of these e-cigarettes was presented to 14 panellists comprising e-cigarette users, and the panellists were asked to puff on the e-cigarettes in a sequential monadic fashion for 10 puffs on each device. They were asked to identify the preferred e-cigarette from the three offered to them.
3 panellists preferred the unprotonated control e-cigarette, and 11 people preferred the acidified samples - 7 preferred the 0.3Meq benzoic acid device and 4 preferred the 0.3Meq levulinic acid device.

### Example 5

A series of tests were conducted using Vype E-pen electronic cigarettes. The "unprotonated nicotine control" devices were loaded with solution containing 1.8% (w/w) nicotine, 25% propylene glycol containing tobacco flavour "B", 25% water and 48.1% glycerol. This solution had a pH of 9.3 indicating nicotine protonation at a level of 1%.

A similar set of devices were prepared wherein 0.41% w/w (0.3Meq to nicotine) benzoic acid was added to the formulation, with the glycerol content was commensurately reduced to 47.7% (w/w). This solution had a pH of 7.7 indicating nicotine protonation at a level of 28%.

A third set of devices were prepared wherein 0.39% w/w (0.3Meq to nicotine) levulinic acid was added to the formulation, with the glycerol content was commensurately adjusted to 47.8% (w/w). This solution had a pH of 7.4 indicating nicotine protonation at a level of 41%.

One each of these e-cigarettes was presented to 11 panellists comprising e-cigarette users, and the panellists were asked to puff on the e-cigarettes in a sequential monadic fashion for 10 puffs on each device. They were asked to identify the preferred e-cigarette from the three offered to them.
4 panellists preferred the unprotonated control e-cigarette, and 7 people preferred the acidified samples - 4 preferred the 0.3Meq benzoic acid device and 3 preferred the 0.3Meq levulinic acid device.

### Example 6

A series of tests were conducted using Vype E-stick electronic cigarettes. The "unprotonated nicotine control" devices were loaded with solution containing 4% (w/w) nicotine, 25% propylene glycol containing a cherry flavour, 9% water and 62% glycerol. This solution had a pH of 8.3 indicating nicotine protonation at a level of 7 %.

A similar set of devices were prepared wherein 1.2% w/w (0.4Meq to nicotine) benzoic acid was added to the formulation, with the glycerol content was commensurately reduced to 60.8% (w/w). This solution had a pH of 7.4 indicating nicotine protonation at a level of 41%.

A third set of devices were prepared wherein 1.15% w/w (0.4Meq to nicotine) levulinic acid was added to the formulation, with the glycerol content was commensurately adjusted to 60.9% (w/w). This solution had a pH of 6.9 indicating nicotine protonation at a level of 68 %.

One each of these e-cigarettes was presented to 11 panellists comprising e-cigarette users, and the panellists were asked to puff on the e-cigarettes in a sequential monadic fashion for 10 puffs on each device. They were asked to identify the preferred e-cigarette from the three offered to them.
1 panellist preferred the unprotonated control e-cigarette, and 10 people preferred the acidified samples - 6 preferred the 0.4Meq benzoic acid device and 4 preferred the 0.4Meq levulinic acid device.

### Example 7

A series of tests were conducted using Vype E-stick electronic cigarettes. The "unprotonated nicotine control" devices were loaded with solution containing 4% (w/w) nicotine, 36.5% propylene glycol containing a mint flavour, 9% water and 50.5% glycerol. This solution had a pH of 9.6 indicating nicotine protonation at a level of <1%.

A similar set of devices were prepared wherein 1.2% w/w (0.4Meq to nicotine) benzoic acid was added to the formulation, with the glycerol content was commensurately reduced to 49.3% (w/w). This solution had a pH of 7.3 indicating nicotine protonation at a level of 51%.

A third set of devices were prepared wherein 1.15% w/w (0.4Meq to nicotine) levulinic acid was added to the formulation, with the glycerol content was commensurately adjusted to 49.35% (w/w). This solution had a pH of 6.8 indicating nicotine protonation at a level of 73%.

One each of these e-cigarettes was presented to 11 panellists comprising e-cigarette users, and the panellists were asked to puff on the e-cigarettes in a sequential monadic fashion for 10 puffs on each device. They were asked to identify the preferred e-cigarette from the three offered to them.
2 panellist preferred the unprotonated control e-cigarette, and 9 people preferred the acidified samples - 5 preferred the 0.4Meq benzoic acid device and 4 preferred the 0.4Meq levulinic acid device.

### Example 8

A series of tests were conducted using Vype E-pen electronic cigarettes. The devices were loaded with the following solutions
A - 1.86% w/w nicotine, 0.42% w/w benzoic acid (~0.3Meq to nicotine), 47.72% w/w glycerol, 25% w/w water, 19.5% w/w propylene glycol and 5.5% w/w flavour
B - 1.86% w/w nicotine, 0.42% w/w benzoic acid (~0.3Meq to nicotine), 47.72% w/w glycerol, 25% w/w water, 13% w/w propylene glycol and 12% w/w flavour
C - 1.86% w/w nicotine, 0.42% w/w benzoic acid (~0.3Meq to nicotine), 37.22% w/w glycerol, 25% w/w water, 30% w/w propylene glycol and 5.5% w/w flavour

Various modifications and variations of the present invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry or related fields are intended to be within the scope of the following claims.

Further aspects of the invention are provided in the following numbered paragraphs:
1. A nicotine solution comprising
   (i) a carrier;
   (ii) nicotine in unprotonated form and in protonated form; and
   (iii) one or more acids, wherein at least benzoic acid, levulinic acid or a mixture thereof is present; and
      wherein the total content of acid present in the solution is no greater than 0.6 mole equivalents based on the nicotine.
2. A nicotine solution according to paragraph 1 further comprising water.
3. A nicotine solution according to paragraph 1 or 2 wherein at least benzoic acid is present.
4. A nicotine solution according to paragraph 1, 2 or 3 containing acid selected from the group consisting of benzoic acid.
5. A nicotine solution according to any one of paragraphs 1 to 3 containing acid selected from the group consisting of levulinic acid.
6. A nicotine solution according to any one of paragraphs 1 to 5 wherein the total content of acid present in the solution is no greater than 0.5 mole equivalents based on the nicotine.
7. A nicotine solution according to any one of paragraphs 1 to 6 wherein the total content of acid present in the solution is no less than 0.2 mole equivalents based on the nicotine.
8. A nicotine solution according to any one of paragraphs 1 to 8 wherein the amount of benzoic acid present in the solution is no less than 0.2 mole equivalents based on the nicotine.
9. A nicotine solution according to any one of paragraphs 1 to 8 wherein the combined amount of benzoic acid and levulinic acid present in the solution is from 0.2 to 0.4 mole equivalents based on the nicotine.
10. A nicotine solution according to any one of paragraphs 1 to 9 wherein the amount of benzoic acid present in the solution is from 0.2 to 0.4 mole equivalents based on the nicotine.
11. A nicotine solution according to any one of paragraphs 1 to 10 comprising nicotine in an amount of no greater than 2 wt% based on the total weight of the solution.
12. A nicotine solution according to any one of paragraphs 1 to 10 comprising nicotine in an amount of no greater than 1.8 wt% based on the total weight of the solution.
13. A nicotine solution according to any one of paragraphs 1 to 12 containing acid selected from the group consisting of levulinic acid and comprising nicotine in an amount of no greater than 1.8 wt% based on the total weight of the solution.
14. A nicotine solution according to any one of paragraphs 1 to 13 wherein the carrier is a solvent.
15. A nicotine solution according to paragraph 14 wherein the solvent is selected from glycerol, propylene glycol and mixtures thereof.
16. A contained nicotine solution comprising
   (a) a container; and
   (b) a nicotine solution, comprising
      (i) a carrier;
      (ii) nicotine in unprotonated form and in protonated form; and
      (iii) one or more acids, wherein at least benzoic acid, levulinic acid or a mixture thereof is present; and
         wherein the total content of acid present in the solution is no greater than 0.6 mole equivalents based on the nicotine.
17. A contained nicotine solution according to paragraph 16 wherein the nicotine solution further comprises water.
18. A contained nicotine solution according to paragraph 17 wherein the container is configured for engagement with an electronic vapour provision system.
19. A contained nicotine solution according to paragraph 16, 17 or 18 wherein the nicotine solution is as defined in any one of paragraphs 2 to 15.
20. An electronic vapour provision system comprising:
   a vaporiser for vaporising liquid for inhalation by a user of the electronic vapour provision system;
   a power supply comprising a cell or battery for supplying power to the vaporiser a nicotine solution, comprising
      (i) a carrier;
      (ii) nicotine in unprotonated form and in protonated form; and
      (iii) one or more acids, wherein at least benzoic acid, levulinic acid or a mixture thereof is present; and
         wherein the total content of acid present in the solution is no greater than 0.6 mole equivalents based on the nicotine.
21. An electronic vapour provision system according to paragraph 20 wherein the nicotine solution is as defined in any one of paragraphs 2 to 15.
22. A process for improving the sensory properties of a vaporised nicotine solution, the process comprising the steps of
   (a) providing a nicotine solution comprising
      (i) a carrier;
      (ii) nicotine in unprotonated form and in protonated form; and
      (iii) one or more acids, wherein at least benzoic acid, levulinic acid or a mixture thereof is present; and
         wherein the total content of acid present in the solution is no greater than 0.6 mole equivalents based on the nicotine;
   (b) vaporising the nicotine solution
23. A process according to paragraph 22 wherein the nicotine solution is as defined in any one of paragraphs 2 to 14.
24. Use of one or more acids for improving sensory properties of a vaporised nicotine solution, wherein the nicotine solution comprises
   (i) a carrier;
   (ii) nicotine in unprotonated form and in protonated form; and
   (iii) one or more acids, wherein at least benzoic acid, levulinic acid or a mixture thereof is present; and
wherein the total content of acid present in the solution is no greater than 0.6 mole equivalents based on the nicotine.

## Claims

1. A nicotine solution, comprising:
(i) a carrier;
(ii) nicotine in unprotonated form and in protonated form; and
(iii) one or more acids, wherein at least benzoic acid is present.

2. The nicotine solution according to claim 1, further comprising water.

3. The nicotine solution according to claim 1 or claim 2, wherein the amount of benzoic acid present in the solution is no greater than 0.6 mole equivalents based on the nicotine.

4. The nicotine solution according to any one of claims 1 to 3, wherein the amount of benzoic acid present in the solution is no less than 0.2 mole equivalents based on the nicotine.

5. The nicotine solution according to any one of claims 1 to 4, wherein the total content of acid present in the solution is no greater than 0.6 mole equivalents based on the nicotine.

6. The nicotine solution according to any one of claims 1 to 5, wherein the nicotine solution further comprises acetic acid or succinic acid in an amount of no greater than 0.1 mole equivalents based on the nicotine.

7. The nicotine solution according to any one of claims 1 to 6, wherein 1 to 80 wt% of the nicotine present in the solution is in protonated form.

8. The nicotine solution according to any one of claims 1 to 7, wherein the nicotine solution may also comprise flavouring components.

9. The nicotine solution according to any one of claims 1 to 8, wherein the nicotine solution comprises nicotine in an amount of no greater than 2 wt% based on the total weight of the solution.

10. The nicotine solution according to any one of claims 1 to 9, wherein the nicotine solution comprises nicotine in an amount of no greater than 1.8 wt% based on the total weight of the solution.

11. The nicotine solution according to any one of claims 1 to 10, wherein the carrier is a solvent.

12. The nicotine solution according to claims 11, wherein the solvent is selected from glycerol, propylene glycol and mixtures thereof.
